# EUROPEAN PATENT SPECIFICATION

(11) **EP 3 508 315 B1**
(45) Date of publication and mention of the grant of the patent: **24.03.2021**
(21) Application number: 18397501.0
(22) Date of filing: 03.01.2018
(51) Int. Cl.: B27D 1/04, B27G 1/00, B27L 5/00, B27L 5/08, B07C 5/36, B07C 5/14, B07C 5/342, G01N 33/46, G01N 21/898, G06T 7/64

(54) **A METHOD AND A SYSTEM FOR MANUFACTURING PLYWOOD**
VERFAHREN UND SYSTEM ZUR HERSTELLUNG VON SPERRHOLZ
PROCÉDÉ ET SYSTÈME DE FABRICATION DE CONTREPLAQUÉ

(43) Date of publication of application: 10.07.2019
(73) Proprietor: UPM Plywood Oy, 15140 Lahti (FI)
(72) Inventor: SORJONEN, Mika, 81100 KONTIOLAHTI (FI); VALKONEN, Tomi, 53500 LAPPEENRANTA (FI); HÄNNINEN, Joonas, 80140 JOENSUU (FI)
(74) Representative: Berggren Oy, Tampere

(56) References cited:
- JP-A- S5 899 737
- US-A- 3 560 096
- US-A- 4 375 921
- US-A- 4 602 663
- US-A- 4 934 228

## Description

### Technical field

The invention relates to a method according to the preamble of claim 1 and a system according to the preamble of claim 11 for manufacturing plywood. Such a method is known from US 4 934 228 A.

### Background

Plywood is manufactured by laying sheets of veneers on top of each other, adhesive in between, and pressing them to form a plywood panel. In order to manufacture high-quality plywood, in particular the surface layers should be free from defects. Veneer is commonly produced by peeling from a log, typically called a peeler log. A veneer lathe is used for the purpose. However, veneer lathes are known to produce various kinds of peeling defects to the veneer.

The presence of certain type of peeling defects bears evidence of that the veneer lathe, in particular the knife gap thereof, has become dirty. Correspondingly, the veneer lathe will produce defected veneer as long as the knife gap is not cleaned or the dirt will run out by itself. Such defects typically have a shape of a protrusion or a depression, which extends in a crosswise direction of the veneer, i.e. in a direction perpendicular to a grain orientation. Such defects may be observable by an operator, provided that he/she is present. Moreover, if the operator operates multiple lathes, the detection becomes hard. Furthermore, manual observation of such defects may be so slow, that the entire log is peeled before the presence of a peeling effect is observed and the knife gap is cleaned. This results in quite some waste, since the defected veneer may not necessarily be used. However, efficient production of plywood requires minimizing the amount of waste. Furthermore, in high-quality plywood, also the middle veneer layers should be free from peeling defects in order to diminish variation of the strength from one plywood panel to another.

### Summary

It has been found that amount of waste can be significantly reduced, when the presence of peeling defects is done automatically. This has at least three effects. First, when the process of determining the presence of a peeling defect is automatic, it is also much faster than a manual method. Thus, one can react much more rapidly to the defects and minimize the production of waste. In addition, a greater portion of the veneer sheets may be used as surface veneers. Second, the accuracy of an imaging device and a processor, in combination, may be so high that a height or a depth of the defect can be sufficiently accurately determined. Thus, it is also possible to allow the production of veneers with small (i.e. shallow or low) peeling defects. Thus, not all defected veneers need to be wasted. Third, in the process, the variation of the quality of the veneers is also diminished, whereby the variation of some mechanical parameters, including strength, may also diminished. It has been found that the process can be automated by using an imaging device. The method according to the invention is defined in claim 1. A corresponding system according to the invention is defined in claim 11.

### Brief description of the drawings

- Fig. 1a: shows a method for manufacturing a plywood panel,
- Fig. 1b: shows a method for manufacturing a plywood panel,
- Fig. 2a: shows a veneer lathe and a method for peeling a peeler log,
- Fig. 2b: shows a part of a veneer lathe and a method for peeling a peeler log,
- Fig. 3a: shows, in a side view, a part of a veneer lathe and an imaging device for imaging a surface of the veneer,
- Fig. 3b: shows, in a side view, a part of a veneer lathe and an imaging device for imaging a surface of the veneer,
- Fig. 4a: shows, in an top view, a veneer lathe and an imaging device for imaging a surface of the veneer,
- Fig. 4b: shows, in an end view, a veneer the imaging device of Fig. 4a,
- Fig. 5a: shows, in an top view, a veneer having a peeling defect,
- Fig. 5b: shows, in an end view, a veneer having a low peeling defect, i.e. a defect of which height is less than a tolerance value,
- Fig. 5c: shows, in an end view, a veneer having a high peeling defect, i.e. a defect of which height is more than a tolerance value,
- Fig. 6a: shows, in a side view, a plywood panel that has not been sanded,
- Fig. 6b: shows, in a side view, the plywood panel of Fig. 6a after sanding,
- Fig. 7a: shows, in a side view, a method for imaging a surface of a veneer, the surface being free from peeling defects,
- Fig. 7b: shows, in a side view, the method of Fig. 7a, when the surface of the veneer comprises a peeling defect, and
- Fig. 7c: shows an image of a surface of the veneer, as imaged with the method of Figs. 7a and 7b.

In the figures, AX denotes the axial direction of the peeler log, i.e. the grain direction of the peeler log. The direction Sy denotes the crosswise direction of the veneer. The crosswise direction Sy is perpendicular to the grain direction AX, and a surface of the veneer extends in the axial and the crosswise directions. The direction Sz denotes the direction of thickness of veneer, e.g. when the veneer is arranged substantially horizontally. Correspondingly, in a typical method, Sz is substantially vertical.

### Detailed description

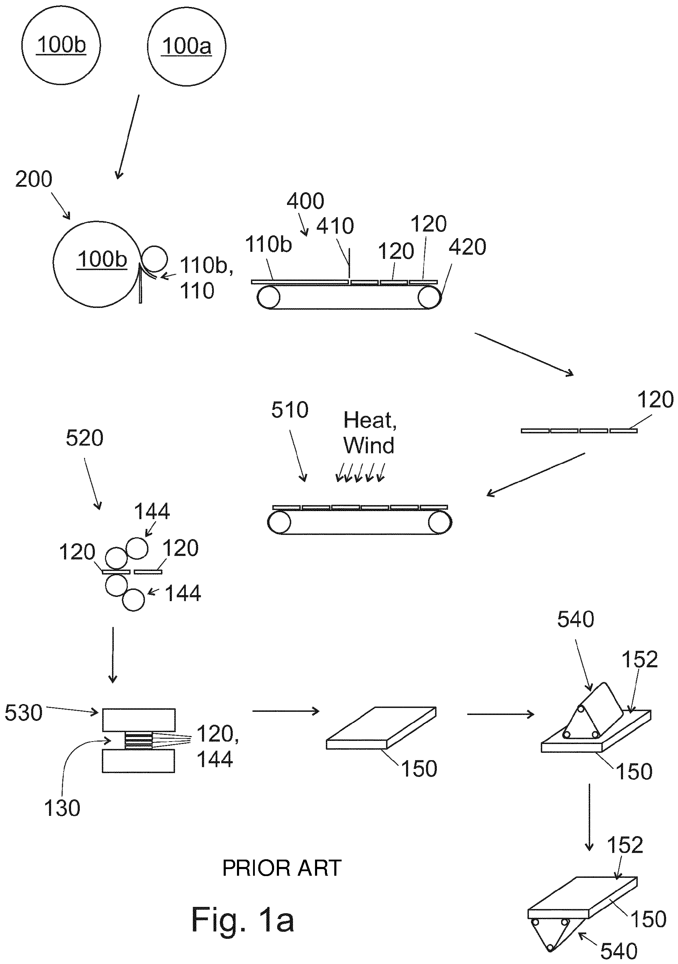

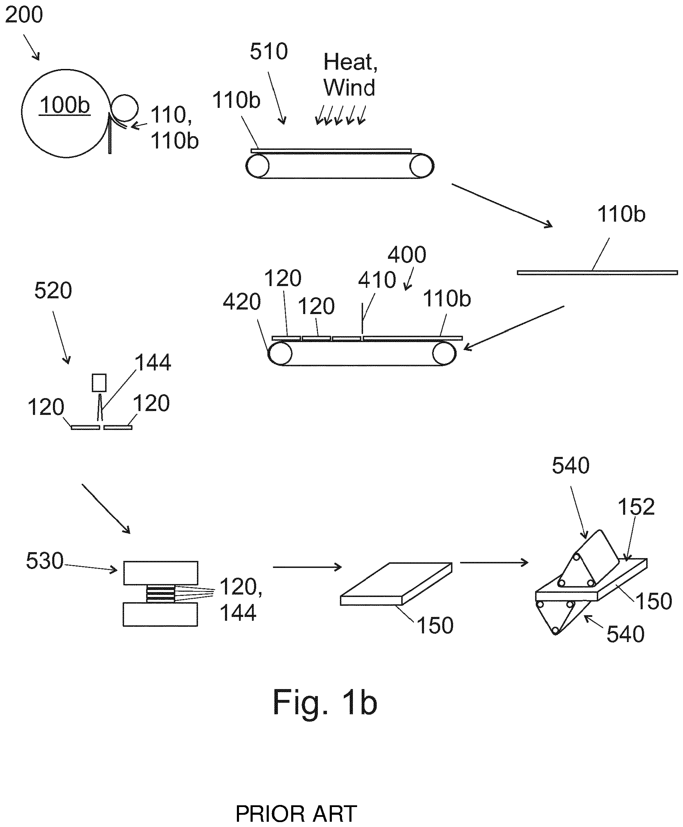

Figs. 1a and 1b indicate a process for manufacturing a plywood panel 150 as known from prior art. Dried veneer sheets 120 are commonly used to manufacture plywood. In order the manufacture veneer 110 and/or veneer sheets 120 from a peeler log 100, veneer is typically peeled using a veneer lathe 200.

Parts of a process for manufacturing plywood is shown in Figs. 1a and 1b. Referring to Fig. 1a, after (i) soaking or steaming and (ii) de-barking, wood is cut into logs 100, 100a, 100b. In general, all logs 100, 100a, 100b are treated in a similar manner. The logs 100, 100a, 100b, i.e. the peeler logs, will be peeled to form veneer 110, 110a, 110b. However, only such veneer 110, of which quality is sufficiently high, will be used for manufacturing plywood panel. The rest may be wasted. For example, a secondary peeler log 100b may produce veneers sufficiently free from peeling defects, whereby the veneer sheets 120 and the plywood panel 150 may be manufactured as known, and indicated in Figs. 1a and 1b. However, a primary peeler log 100a may produce a defected veneer 110a.

Thus, a (secondary) veneer 110b is made from a (secondary) peeler log 100b. A veneer lathe 200 is used for peeling the veneer 110b from the peeler log 100b. Thereafter, the veneer 110b may be cut to veneer sheets 120. The veneers 110 are cut in a cutting apparatus 400 having a blade 410 for cutting (i.e. clipping) the veneer 110 into veneer sheets. Thereafter, at least some of the veneer sheets 120 are dried in a drier 510.

Thereafter, adhesive 144 is arranged onto at least one side of at least some of the dry veneers sheets 120. Adhesive 144 may be applied in an applicator 520. Thereafter, a stack of dry veneer sheets 120 and adhesive 144 is pressed, in a press 530, to form a plywood panel 150. The plywood panel 150 is typically thereafter sanded with a sanding apparatus 540. Typically both sides of the panel are sanded. They may be sanded subsequently, as in Fig. 1a, or simultaneously, as in Fig. 1b, which is more common. The plywood may be also finished e.g. by sawing the edges and/or by coating the plywood panel 150. As indicated in Fig. 1a, the veneer sheets 120, having been clipped from the veneer 110, can be dried. As indicated in Fig. 1b, the veneer 110 can be dried before clipping into veneer sheets 120. In principle, both the veneer 110 and the sheets 120 could be dried.

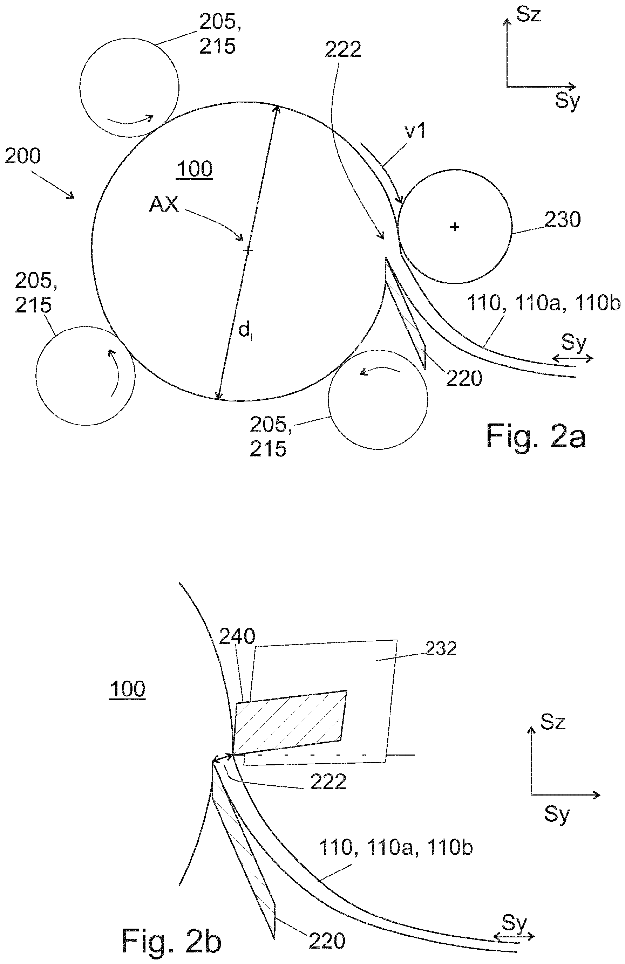

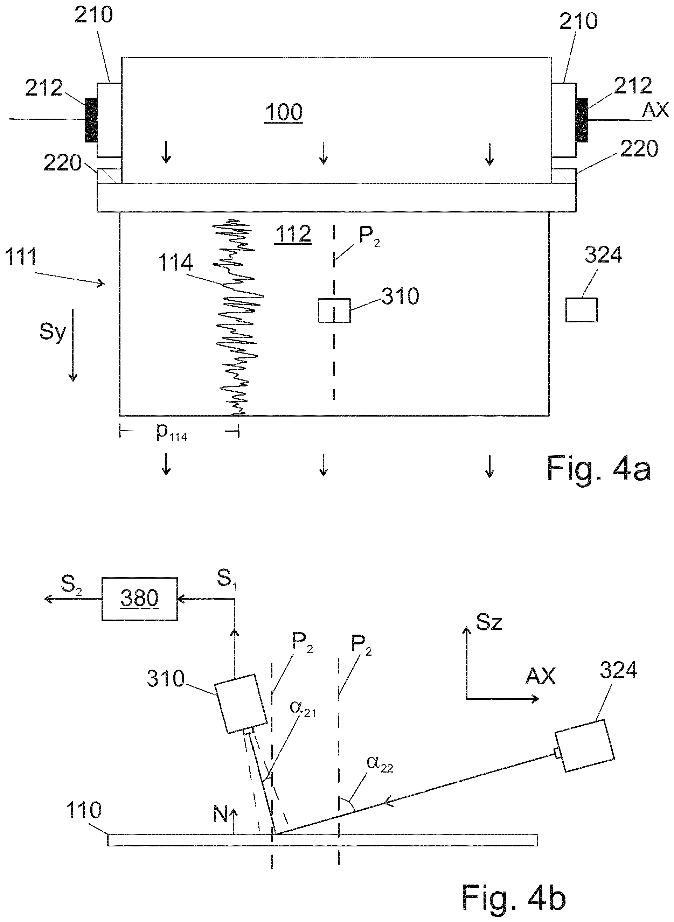

Veneer 110 for plywood may be produced by a rotary method by using a veneer lathe 200. Referring to Figs. 2a and 2b, a veneer lathe 200 in general comprises a rotor 205 for rotating the peeler log 100. The rotor 205 may comprise a lathe chuck 210 (or a pair of lathe chucks 210) for rotating the peeler log 100 from an end of the peeler log, as indicated in Fig. 4a. A lathe chuck 210 is sometimes referred to as a spindle. In the alternative, as indicated in Fig. 2a, the rotor 205 may comprise support rolls 215 configured to rotate the peeler log 100 from such an outer surface of the peeler log 100 that has a normal parallel to the radial direction of the peeler log 100. A lathe 200 without a lathe chuck 210 may be referred to as a spindless lathe 200 or a chuckless lathe 200.

The veneer lathe 200 further comprises a knife 220 for peeling veneer 110 from the peeler log 100, and nose bar (230, 240) configured to press the peeler log 100. The peeler log 100 is configured to rotate about a first axis of rotation AX. The peeler log 100 is arranged in the lathe 200 such that the grain orientation of the peeler log is parallel to the axis of rotation AX. The lathe chuck 210 (if present, or the pair of lathe chucks 210) is configured to rotate about the first axis of rotation AX. In such a lathe 200, a knife gap

222 is left in between the nose bar (230, 240) and the knife 220. As an alternative term for the knife gap, the term exit gap is sometimes used. The veneer 110 is configured to exit through the knife gap 222.

Fig. 2a shows a veneer lathe 200 having a roller nose bar 230. In such a lathe 200, the roller nose bar 230 rotates while peeling the peeler log 100. Fig. 2b shows a veneer lathe 200 having a fixed nose bar 240. Typically the lathe 200 comprises a pressure head 232, which is configured to press the roller nose bar 230 or the fixed nose bar 240 against the peeler log 100. When peeling the peeler log 100, the peeler log 100 rotates about the axis of rotation AX with a first peripheral velocity v1 as indicated in Fig. 2a. The peripheral velocity v1 is, by definition, the radius of the peeler log 100 multiplied by an angular velocity of the peeler log 100. Both types of veneer lathes produce a large piece of veneer 110, whereby the veneer 110 is later cut to veneer sheets 120, optionally after drying, as indicated above.

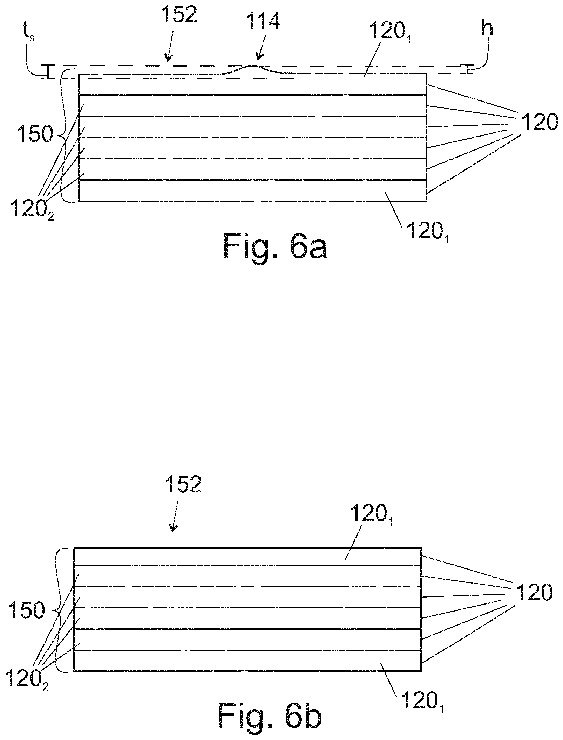

Typically, some of the veneer sheets 120 are used as surface veneer sheets 120₁ of a plywood panel 150, and some are used mid-veneer sheets 120₂ of a plywood panel 150, as indicated in Figs. 6a and 6b. In general, the quality of a surface veneer sheet 120₁ must be high, because the surface veneer sheet 120₁ forms a surface 152 of the plywood panel 150, the surface 152 being typically visible for a user. Thus, the veneer sheets 120, as produced, are typically classified according to their quality to surface veneers 120₁ and mid-veneers 120₂. Typically high quality surface veneers 120₁ are obtained from surface parts of the peeler log 100. The inner parts (i.e. core) of the peeler log 100 often include colouring defects or similar, whereby veneer sheets 120 from the inner parts are often not usable as surface veneers 120₁. For these reasons, in general there is a surplus of mid-veneer sheets 120₂, and correspondingly, a shortage of surface veneer sheets 120₁.

However, the quality of the mid-veneers sheets 120₂ should also not be too low. For example, if the mid-veneers sheets 120₂ comprise high or deep large peeling defects 114, e.g. three-dimensional peeling defects 114, this may affect the adhesive bonding between such two veneer layers that are next to each other. For example, a defect 114 that is a protrusion, may squeeze adhesive away from the point of the contact of the protrusion 114 with a neighbouring veneer sheet 120. This may deteriorate the strength of the plywood panel 150, at least if the height or depth of the defect 114 is significant. Also for this reason, it is beneficial to produce veneer 110 free from peeling defects. As reasoned above, this is beneficial also in cases, where the veneer 110 has some other types of defects and is thus used as a mid-veneer 120₂.

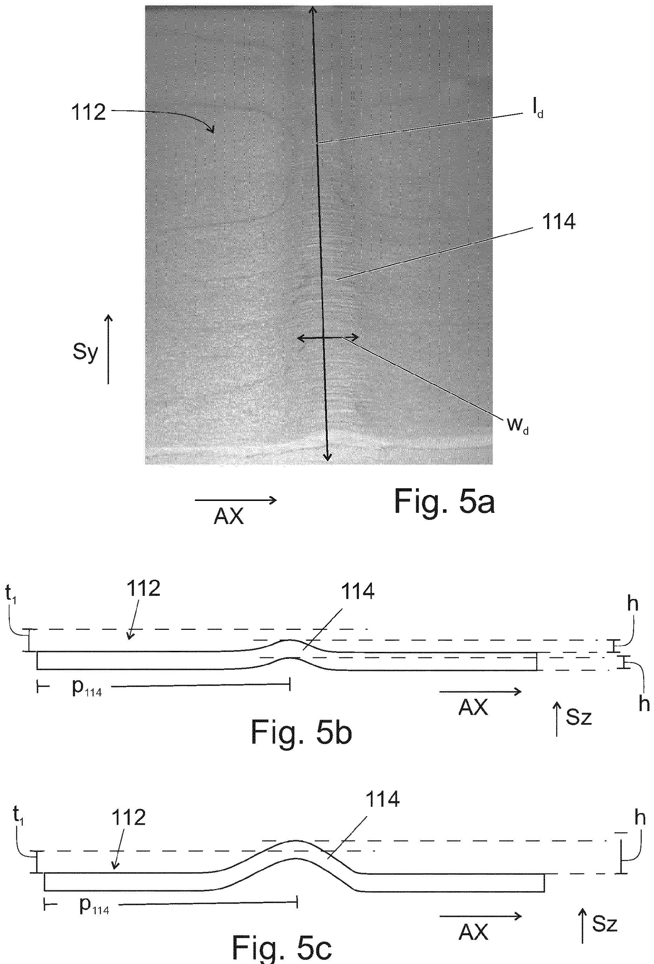

Referring to Figs. 4a, 5a, 5b, and 5c, it has been noticed that sometimes a surface 112 of the veneer 110 comprises a peeling defect 114. In particular, the veneer 110 may comprise a peeling defect 114 having a width in the grain direction AX and extending on the surface 112 of the veneer 110 also in the crosswise direction Sy, which is perpendicular to the grain direction AX. Such a peeling defect is a protrusion or a depression extending in two directions of the surface 112 of the veneer 110. The width W_{d} of such a peeling defect is typically at least 2 mm, such as from 5 mm to 50 mm. The width W_{d} is oriented in the grain direction AX, as indicated in Fig. 5a. Moreover a length I_{d} of such a peeling defect 114 is typically several metres, as the defect 114 is being produced by a dirty lathe 200. The length I_{d} is oriented in the crosswise direction Sy, as indicated in Fig. 5a. Thus, such a peeling defect 114 is a protrusion or a depression extending in the grain direction for at least 2 mm, e.g. from 5 mm to 50 mm, and extending on the surface 112 of the veneer 110 also in the crosswise direction for more than 50 mm, e.g. more than 150 mm. Such a peeling defect 114 is referred to as a three-dimensional peeling defect 114. Many other defects, such as lathe checks are substantially one dimensional. As indicated in Figs. 5b, 5c, and 6, a height or a depth of such a peeling defect 114 is denoted by h.

Such a three-dimensional peeling defect 114 is typically a result of the knife gap 222 being dirty. For example, if de-barking of the logs 100 is not perfect, bark may enter the knife gap 222 and be responsible for such a three-dimensional peeling defect.

Such three-dimensional peeling defects are not uncommon. It has been found that typically about 3 to 15 percent of all veneers 110 comprise such peeling defects 114. Moreover, typically such a peeling defect extends throughout a veneer 110 in the longitudinal direction Sy. Thus, about 3 % to 15 % of all the veneer sheets comprise such a three-dimensional peeling defect 114 for this reason. Thus, from a number of logs, typically about 85 % to 97 % are peeled in such a way that a secondary veneer 110b, which is free from three-dimensional peeling defects, can be produced. However, typically from about 3 % to 15 % are peeled in such a way that a primary veneer 110a, which comprises a three-dimensional peeling defect, is produced.

As motivated above, even if a secondary veneer 110b, peeled from a secondary log 100b, is free from three-dimensional peeling defects, a primary veneer 110a, peeled from a primary log 100a, may comprise a peeling defect 114, such as a three-dimensional peeling defect 114. The problem is typically more significant for hardwood than for softwood. Moreover, the quality requirements for plywood made of hardwood are typically more stringent that the quality requirements for plywood made of softwood, because of typical uses of hardwood plywood. Thus, in an embodiment, the primary log 100a and the secondary log 100b are logs of hardwood, such a birch. The method is applicable also for softwood. Thus, in an embodiment, the primary log 100a and the secondary log 100b are logs of softwood, such as spruce. As is conventional, the term softwood refers to wood from coniferous trees, such as spruce, pine, fir, and hemlock. The term hardwood refers to wood from angiosperms, such as ash, aspen, basswood, birch, cherry, hickory, mahogany, maple, oak, poplar, lauan, teak, rosewood, okume, and meranti; in particular birch.

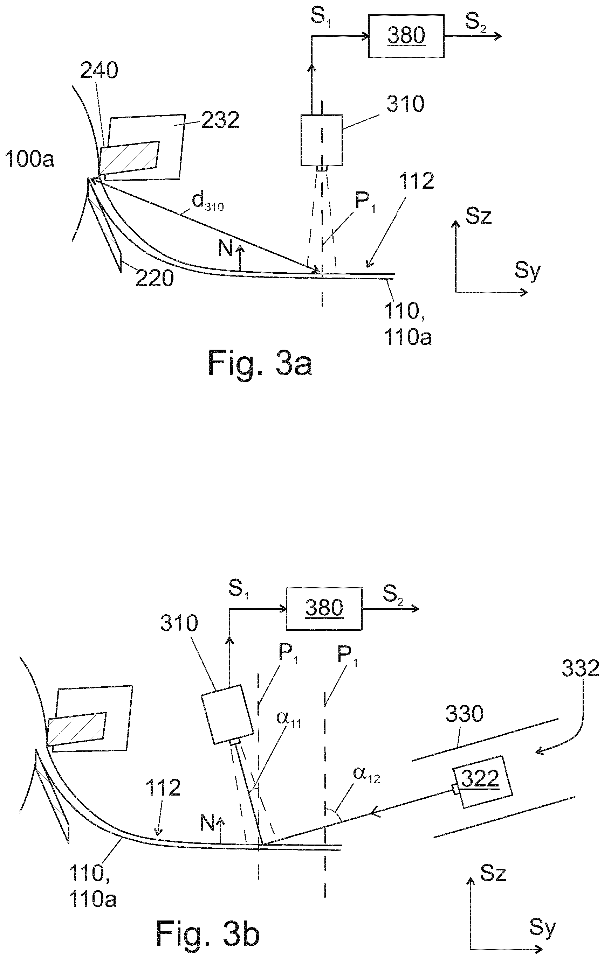

Plywood that is made from hardwood is typically used in such solutions that require high mechanical strength. Such solutions include floorings of vehicles, e.g. trucks and/or truck trailers; as well as high quality thermal insulators. As an example of thermal insulators, in LNG (liquefied natural gas) tankers, the tank is commonly thermally isolated from the walls of the tanker by an insulator having walls made of hardwood plywood, e.g. birch plywood. The space in between the plywood walls is typically filled with suitable thermal insulator. However, the strength and quality requirements for the plywood in this application are high. Therefore, for example plywood intended for use in LNG tankers must not have, even as mid-veneer sheets 120₂, veneer sheets that comprise three-dimensional peeling defects, of which height or depth exceeds a tolerance value, e.g. 0.5 mm or 0.6 mm. Referring to Figs. 3a to 4b, to diminish the amount of veneer not usable for plywood production, an imaging device 310 is used to detect the presence of peeling defect 114 in the veneer 110. The imaging device 310 is preferably arranged at such a location, that the system, or a user of the system, can react to the defects before an entire peeler log 100 has been peeled.

Referring to Fig. 3a, an embodiment of the system comprises an imaging device 310 configured to form a first signal S₁ indicative of a profile of the surface 112 of the veneer 110. Moreover, when the surface 112 actually comprises a peeling defect 114, the veneer may be referred to as a primary veneer, as indicated above. Thus, a corresponding method comprises imaging a surface 112 of the primary veneer 110a with an imaging device 310 to form a first signal S₁ indicative of a profile of the surface 112 of the primary veneer 110a. Preferably, the imaging device 310 is an optoelectronic imaging device. Preferably, the imaging device 310 is configured to form the first signal S₁ by imaging the surface 112 at a wavelength or wavelengths, wherein the wavelength or at least one of the wavelengths is at the infra-red, optical, or ultra-violet regime. The imaging device 310 may be e.g. a digital camera configured to take a picture of the surface 112, and the first signal S₁ may comprise such information that the picture can be reconstructed from the signal S₁. The digital camera may configured to form the first signal S₁ by receiving light at the optical and/or infra-red wavelength regime. Thus, a thermographic camera is an example of a digital camera.

The method further comprises, by using the first signal S₁, determining that the surface 112 of the primary veneer 110a has a peeling defect 114. Some preferable ways for the determination will be discussed below. Moreover, an embodiment of the method comprises generating a second signal S₂ indicative of the surface 112 of the primary veneer 110a having the peeling defect 114. How such a second signal S₂ can be used will be discussed below. That the surface 112 of the primary veneer 110a has a peeling defect 114 is preferably determined using a processor 380. Correspondingly the system comprises a processor 380 configured to determine, by using the first signal S₁, that the surface 112 of the veneer 110 has a peeling defect 114. In an embodiment, the processor 380 is also configured to generate a second signal S₂ indicative of the surface 112 of the veneer 110 having a peeling defect 114. Correspondingly, an embodiment of the method comprises determining that the surface 112 of the primary veneer 110a has a peeling defect 114 by means of the processor 380 and by using the first signal S₁.

As indicated above, the method is particularly suitable for determining the presence of a three-dimensional peeling defect 114. Therefore, an embodiment comprises by using the first signal S₁, determining that the surface 112 of the primary veneer 110a has a three-dimensional peeling defect 114. An embodiment comprises generating a second signal S₂ indicative of the surface 112 of the primary veneer 110a having a three-dimensional peeling defect 114. The measures of a three-dimensional peeling defect 114 have been discussed above, and apply in these embodiments.

Determining that the surface 112 of the primary veneer 110a has a three-dimensional peeling defect 114 may be hard based on only one image of the surface 112. One image of the surface 112 may e.g. indicate presence of some kind of defect or dirt. Moreover, it has been noticed that, if determined based on one image only, risk of determining that the surface 112 of the veneer 110 has a peeling defect 114 also in the case, where the surface 112 actually is free from peeling defects increases. Therefore, preferably the method comprises imaging the surface 112 of the primary veneer 110a with the imaging device 310 at multiple instances of time to form such a first signal S₁ that is indicative of a profile of the surface of the veneer in two dimensions that are perpendicular to a thickness of the primary veneer 110a. Moreover, preferably the method comprises using the signal S₁ to form information indicative of the profile of the surface 112 of the primary veneer 110a at multiple instances of time, and by using this information, determining the presence of only one three-dimensional peeling defect 114.

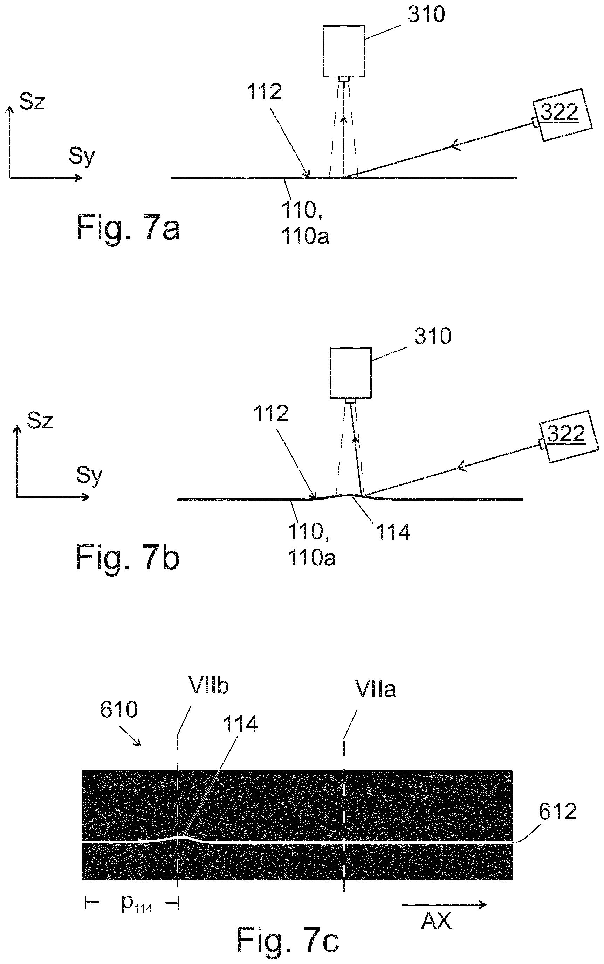

Thus, in an embodiment of a system, a processor 380 is configured to determine, by using the first signal S₁, that the surface 112 of the veneer 110 has a three-dimensional peeling defect 114. Moreover, the processor 380 is configured to receive such a first signal S₁. Such a first signal S1 is indicative of a profile of the surface 112 at multiple instances of time. Moreover, such a first signal S1 is indicative a profile of the surface 112 of the veneer 110 in two mutually perpendicular dimensions that are perpendicular to a thickness of the veneer 110. However, the signal S1 needs not to be indicative of two-dimensional profile at multiple instance of time. For example, images as indicated in Fig. 7c are only indicative of a profile in one dimension (i.e. the direction AX). However, when multiple such images are taken subsequently, the images, in combination, are indicative of a profile in two-dimensions, i.e. a three-dimensional defect 114. Moreover, in an embodiment, the imaging device 310 is configured to form such a first signal S₁. Moreover, in an embodiment, the imaging device 310 is configured to send such a first signal S₁ to the processor 380.

Referring to Fig. 3a, a system for manufacturing plywood panel 150 comprises an imaging device 310, such as a digital camera, as discussed above. However, it has been found that the three-dimensional peeling defects 114 are not easily visible, as indicated in Fig. 5a. Therefore, an embodiment of a system comprises a light source (322, 324), such as a primary light emitting device 322 and/or a secondary light emitting device 324. The light source 322, 324 is configured to illuminate such a part of the surface 112 that the imaging device 310 images. Correspondingly, an embodiment of the method comprises illuminating such a part of the surface 112 that is imaged by the imaging device 310. This improves the visibility of the three-dimensional peeling defects 114. Moreover, preferably, the primary light emitting device 322 and/or a secondary light emitting device 324 is/are configured to emit electromagnetic radiation (e.g. infra-red, optical, or ultra-violet radiation) at such a wavelength, that, when received by the imaging device 310, the imaging device 310 is configured to form the first signal S1 based on the received radiation at the wavelength.

Two ways of illuminating have been found particularly suitable for the purpose. These ways of illuminating are not mutually exclusive. The first of these ways is indicated in Fig. 3b, and the second way is indicated in Figs. 4a and 4b.

Referring to Fig. 3b, an embodiment of the system comprises a primary light emitting device 322. In the embodiment of Fig. 3b, the imaging device 310, such as a camera, is configured to detect light reflected from the surface 112 of the veneer 110 (or primary veneer 110a) at a first primary angle α₁₁ relative to a first plane P₁ comprising a normal N of the surface 112 of the veneer and a grain direction AX of the veneer 100 (or primary veneer 110a). In other words, a normal of the first plane P₁ is parallel to the crosswise direction Sy (i.e. a longitudinal edge 111 of the veneer; see Fig. 4a for the longitudinal edge 111). Two such first planes P₁ are indicated in Fig. 3b. As indicated in Fig. 3b, the first primary angle α₁₁ is left in between the first plane P₁ and a straight line formed in the central axis of a field of view of the imaging device 310. Correspondingly, in an embodiment, the first primary angle α₁₁ is left in between the first plane P₁ and a straight line from an optical aperture of the imaging device 310 to a centre of the area imaged by the imaging device 310. In Fig. 3a such a first primary angle α₁₁ is zero, since the aforementioned straight line is parallel to the normal N of the surface 112. Thus, the first primary angle α₁₁ may be zero.

In the embodiment of Fig. 3b, the primary light emitting device 322 is configured to emit light to such a part of the surface 112 of the veneer 110 that is imaged by the imaging device 310 and at a second primary angle α₁₂ relative to the first plane P₁ comprising the normal N of the surface 112 of the veneer 110 and the grain direction AX of the veneer 110. In an embodiment, the second primary angle α₁₂ is left in between the first plane P₁ and a straight line from the primary light emitting device 322 to a centre of the area imaged by the imaging device 310. In an embodiment, the primary light emitting device 322 comprises a laser. A laser has the effect that only a small point or line of the surface 112 is illuminated. Such an effect can, as an alternative to a laser, be achieved by proper lenses. Preferably, an absolute value of a difference between the first primary angle α₁₁ and the second primary angle α₁₂ is at least 15 degrees, such as at least 45 degrees. Herein the first primary angle α₁₁ and the second primary angle α₁₂ are directed angles, i.e. each one of them may be positive or negative. For example, in Fig. 3b, the first primary angle α₁₁ opens anti-clockwise relative to the first plane P₁ and second primary angle α₁₂ opens clockwise relative to the first plane P₁ Therefore, in Fig. 3b, only one of the angles α₁₁, α₁₂ is positive, while the other one is negative. For example, in Fig. 3b, a difference between the first primary directed angle α₁₁ and the second primary directed angle α₁₂ is ±90 degrees, even if the absolute value of α₁₂ is about 75 degrees and the absolute value of α₁₁ is about 15 degrees.

Figures 7a and 7b indicate, how a three-dimensional peeling defect 114 can be observed in such a case. Fig. 7a shows a situation, wherein such a part of the surface 112 that is free from peeling defects 114 is illuminated by the primary light emitting device 322. As indicated in Fig. 7a, the illuminated spot (or line) is imaged, by the imaging device 310. Thus, the illuminated spot is seen at a first position in the direction Sy that is perpendicular to the grain direction AX and along the surface 112. Fig. 7b shows a situation, wherein a three-dimensional peeling defect 114 is illuminated by the primary light emitting device 322. As indicated in Fig. 7b, the illuminated spot is imaged, by the imaging device 310. Moreover, because of the three-dimensional peeling defect 114 being a protrusion (or a depression), the illuminated spot is seen at a second position in the direction Sy that is perpendicular to the grain direction AX and along the surface 112, wherein the second position is different from the first position. As indicated in Figs. 7a and 7b, when a protrusion is seen, the position of the illuminated spot moves towards the primary light emitting device 322. Correspondingly, in case a depression would be seen, the position of the illuminated spot would move away from the primary light emitting device 322 (not shown).

Thus, the primary light emitting device 322 may be turned in such a direction that the illuminated spot moves in the grain direction AX. An illuminated spot shows as a bright spot in the image taken by the imaging device 310. Correspondingly, areas that are not illuminated are shown as dark areas, as in Fig. 7c. When the spot is swept in the grain direction AX, a bright line 612 is seen by the imaging device 310. Such a line is shown in Fig. 7c. Figure 7c shows an image 610 taken by the imaging device 310. Moreover, the image 610 shows a bright line 612. Figure 7c also shows a grain direction AX. Thus, for reasons discussed in connection with Figs. 7a and 7b, the presence, location, and one of height or depth of the three-dimensional peeling defect 114 can be found from the image 610. More specifically, the presence of a non-straight part in the line 612 is indicative of the presence of a three-dimensional peeling defect 114, the position of the non-straight part of the line 612 is indicative of the position of the three-dimensional peeling defect 114, and the amplitude of the non-straight part of the line 612 is indicative of the height or depth of the three-dimensional peeling defect 114. As is evident, instead of having an illuminated spot that is swept along the direction AX, the primary light emitting device 322 may be configured to emit an illuminated line onto the surface 112 of the veneer 110. The illuminated line could be e.g. parallel to the grain direction AX. Moreover, as indicated above, when such images are taken subsequently, the extension of the peeling defect 114 also in the crosswise direction Sy can be detected.

Another way to image the a defect 114 is to illuminate the surface 112 from a side, as indicated in Figs. 4a and 4b. As a three-dimensional peeling defect 114 is a protrusion or a depression, edges of the three-dimensional peeling defect 114 may form a shadow to the image seen by the imaging device 310, when the surface 112 is illuminated from side. Referring to Fig. 4b, in an embodiment, the imaging device 310, is configured to detect light reflected from the surface 112 of the veneer 110 (or primary veneer 110a) at a first secondary angle α₂₁ relative to a second plane P₂ having a normal that is parallel to the grain direction AX. However, the magnitude of the first secondary angle α₂₁ has not been observed to affect significantly the detection of peeling defect 114.

Referring to Figs. 4a and 4b, an embodiment of the system comprises a secondary light emitting device 324, configured to emit light to the surface 112 of the veneer 110. The secondary light emitting device 324 is configured to emit light to such a part of the surface 112 of the veneer 110 that is imaged by the imaging device 310. Moreover, to form shadows to the image, preferably the secondary light emitting device 324 is configured to emit light to the surface 112 of the veneer 110 at a second secondary angle α₂₂ relative to a second plane P₂ comprising a normal N of the surface 112 of the veneer 110 and a direction Sy that is perpendicular to the grain direction AX of the veneer 110. Thus, the second plane P₂ has a normal that is parallel to the grain direction AX. The second secondary angle α₂₂ may be at least 30 degrees, such as at least 45 degrees. The secondary light emitting device 324 may comprise a light emitting diode. In an embodiment, the second secondary angle α₂₂ is left in between the second plane P₂ and a straight line from the secondary light emitting device 324 to a centre of the area imaged by the imaging device 310.

The environment, in which the primary light emitting device 322 and/or the secondary light emitting device 324 is arranged is typically harsh. For example, the environment of the light emitting device 322, 324 may comprise dust and/or moisture. If used without any protection, this typically results in dirt accumulating onto the primary light emitting device 322 and/or the secondary light emitting device 324. Such dirt generates shadows to the illuminated area, which makes it harder to determine the three-dimensional peeling defects 114 optically. Therefore, in an embodiment, the system comprises (i) means 330 for cleaning the primary 322 and/or the secondary 324 light emitting device and/or (ii) means 330 for keeping the primary 322 and/or the secondary 324 light emitting device clean. An example of such a means 330 is shown in Fig. 3b. The means 330 may comprise a blower configured to blow clean gas 332 to the primary light emitting device 322 and/or the secondary light emitting device 324 in order to prevent dirt of the environment from accumulating onto the primary light emitting device 322 and/or the secondary light emitting device 324. The means 330 may comprise a mechanical means, such as a brush, for cleaning the primary 322 and/or the secondary 324 light emitting device. An embodiment of the method comprises cleaning the primary 322 and/or the secondary 324 light emitting device.

When the presence of a peeling defect 114 is determined as indicated in Figs. 7a, 7b, and 7c and discussed above, one image 610 (see Fig. 7c) indicates one of height or depth h of the defect 114 and width wₗ (in the grain direction AX) of the defect 114. However such a single image does not provide information on the length of the defect (in the crosswise direction Sy, see Figs. 4a and 5a). Moreover, also dirt or dust may have a similar effect, whereby there is a risk that a presence of a defect 114 is determined, even if the surface 112 is free from defects.

Also when the presence of a peeling defect 114 is determined as in Figs. 4a and 4b, dirt or dust may produce shadows onto the surface 112 whereby there is a risk that a presence of a defect 114 is determined, even if the surface 112 is free from defects.

In order to reduce the risk of faulty determination of a presence of a defect, an embodiment of the method comprises imaging the surface 112 of the primary veneer 110a with the imaging device 310 at multiple instances of time to form such a first signal S₁ that is indicative of a profile of the surface of the veneer in two dimensions that are perpendicular to a thickness of the primary veneer 110a at these multiple instances of time. In practice, a first number N₁ of images are taken of the surface 112, and the presence of a defect 114 is determined, when a second number N₂ of these images indicates a presence of a defect 114, as detailed above. The second number N₂ is preferably at least two. The first number N1 is preferably at least three, and the second number N₂ is preferably at least two thirds of the first number N₁. For example N₁ may be ten and N₂ may be eight. As indicated above, in an embodiment, the signal S₁ carries (i.e. comprises) information of all the N₁ images.

Since a reasonably accurate measurement technique has been developed for the observation of peeling defects 114, as indicated above, it has also been found that many veneers 110 comprise very small defects in terms on height or depth h. Thus, if a second signal S₂ was generated at each time even a small (i.e. shallow or low) defect 114 is observed, the number of such signals would be excessive. Moreover, those peeling defects 114 do not deteriorate the quality of the plywood panel 150. Therefore, an embodiment comprises, by using the first signal S₁, determining that the surface 112 of the primary veneer 110a has a peeling defect 114 of which height or depth h (see Figs. 5a and 5b) exceeds a first tolerance value l₁. In an embodiment, the second signal S₂ is generated only when the height or depth h of the peeling defect exceeds the first tolerance value l₁. The first tolerance value l₁ may be e.g. at least 0.4 mm, such as from 0.4 mm to 1.0 mm. Preferably, the first tolerance value l₁ is from 0.5 mm to 0.8 mm such as from 0.5 mm to 0.7 mm. This ensures that veneer 110 having sufficiently high quality is passed for the production of plywood panel 150.

Typically, after pressing, the plywood panel 150 is sanded, as indicated in Fig. 1a (see, in particular, the sander 540). Thus, an embodiment of the method comprises, after said pressing the veneer sheet stack 130 to form the plywood panel 150, sanding a surface 152 of the plywood panel 150. Correspondingly, an embodiment of a system comprises a sander 540 configured to sand a surface 152 of the plywood panel 150.

It has been noticed that determining the presence of high or deep three-dimensional peeling defects has synergy with the surface 152 being sanded. To illustrate the synergy, an embodiment of the method comprises after said pressing the veneer sheet stack 130 to form the plywood panel 150, sanding a surface 152 of the plywood panel 150 such that at least a sanding thickness tₛ is sanded off from the surface 152 of the plywood panel 150. Such a sanding thickness tₛ is indicated in Fig. 6. As indicated above, an embodiment of the method comprises by using the first signal S₁, determining that the surface 112 of the primary veneer 110a has a peeling defect 114 of which height or depth h exceeds a first tolerance value l₁. Correspondingly, in such as embodiment, the second signal S₂ is not necessarily generated, when the height or depth h of the peeling defect 114 does not exceed the first tolerance value l₁. In such a case, veneer sheets 120, as cut from the veneer 110a, 110b, which comprise a low or shallow peeling defect 114 will be used for manufacturing the plywood panel 150. Correspondingly, veneer sheets 120, as cut from the veneer 110a, 110b, may comprise peeling defect 114 having a height or depth h that is less than the first tolerance value l₁, i.e. h≤l₁. More precisely, such veneer sheets 120 may be used as a surface veneer sheet 120 of the plywood panel, as indicated in Fig. 6. Herein the surface veneer sheet 120 refers to that one of the veneer sheets that comprises the surface 152 of the plywood panel 150.

Thus, the surface 152 of the plywood panel 150 may comprise a peeling defect 114, of which height or depth h does not exceed the first tolerance value l₁. As indicated in the figure, when the sanding thickness tₛ exceeds the height or depth h of the peeling defect 114, the peeling defect 114 will be completely sanded off from the plywood panel 150. Correspondingly, when the sanding thickness tₛ is greater than or equal to the first tolerance value l₁ (i.e. tₛ ≥ l₁), the surface 152 of the plywood panel 150 may comprise only such peeling defects that are finally sanded off. In such an embodiment, the presence of a low or shallow peeling defect 114 (i.e. wherein h≤l₁) is not necessarily determined, whereby the corresponding veneer proceeds to fabrication of veneer sheets 120. Moreover, the presence of a high or deep peeling defect 114 (i.e. wherein h>l₁) is determined, and such veneer 110a may be removed from the process. In the alternative, such a veneer 110a could be used as a mid-veneer sheet 120, i.e. a veneer sheet 120 that is not a surface veneer sheet 120.

One way to use the second signal S₂ is to generate a warning signal. Thus, a warning signal may be generated when the second signal S₂ is received. The warning signal may be audial or visual. The warning signal may be generated e.g. by illuminating a light, such as a red light. Then the operator of the lathe 200 may act as required. The operator may e.g. stop peeling the primary peeler log 100a. The operator may e.g. clean the veneer lathe 200. In particular, when the warning signal is generated, the response time of the operator to react to such a situation is significantly reduced, which reduces the amount of waste.

In the alternative or in addition, the peeling of the primary peeler log 100a may be automatically stopped, e.g. after generating the second signal S₂. Moreover, the veneer lathe 200 may comprise a cleaning apparatus configured to automatically clean veneer lathe 200, e.g. the knife gap 222 thereof, when the peeling has been stopped. In particular, when the peeling of the primary peeler log 100a is automatically stopped, e.g. after generating the second signal S₂, the production of defected veneer is also stopped. This significantly reduces the amount of waste. It is also possible, that provided that the automatic cleaning does not result in the removal of the three-dimensional peeling defect 114 from the subsequently peeled veneer, the peeling of the subsequent veneer is only stopped. In other words, manual maintenance, e.g. cleaning, is required, provided that the automatic cleaning does not result in a removal of the defect 114.

Moreover, irrespective of whether the maintenance (e.g. cleaning) was only automatic, only manual, or involved both automatic and manual maintenance, the subsequent veneer 110 produced from the same primary peeler log 100a, from which the defected primary veneer 110a was peeled, may be free from peeling defects; at least such peeling defects 114, of which height or depth h does not exceed the aforementioned first tolerance value l₁. In such a case, the subsequent veneer 110 produced from the same primary peeler log 100a may be used for manufacturing the plywood panel 150. Correspondingly, an embodiment of the method comprises, after cleaning the veneer lathe 200, peeling the primary peeler log 100a with the veneer lathe 200 to form subsequent veneer 110 and cutting the subsequent veneer 110 to form veneer sheets 120. The method further comprises drying the subsequent veneer 110 and/or the veneer sheets 120, and using the veneer sheets 120 to manufacture the plywood panel 150, as detailed above.

Since some of the veneer sheets 120 may be used as mid-veneers, it is not necessary to remove, from the production of a plywood panel 150, all such veneer sheets 120 that comprise a peeling defect, even a high or deep peeling defect. In particular, stopping the peeling may not be feasible, if the remaining peeler log is very small. In such a case, the veneer 110 (i.e. primary veneer 110a) obtainable from the remaining peeler log 100 may become so short that only a small percentage thereof can be used to form veneer sheets 120. For example, a remainder of the veneer 110 may be reasonably long in comparison the part of the veneer 110 that can be cut to sheets 120. Therefore, in an embodiment, the peeling of the primary peeler log 100a is stopped only, if the presence of a three-dimensional peeling defect 114 (e.g. a three-dimensional peeling defect 114 having a height or a depth h larger that the limit l₁) is determined at such a point of time that a diameter dₗ (see Fig. 2a) of the primary peeler log 100a is sufficiently large. Correspondingly, an embodiment of the method comprises determining a diameter dₗ of the peeler log, and stopping the peeling of the peeler log 100 only if the diameter dₗ of the peeler log 100 exceeds a second tolerance value l₂. As indicated above, the diameter dₗ of the primary peeler log 100a may be determined so that the determined diameter dₗ of the primary peeler log 100a corresponds to the diameter dₗ of the primary peeler log 100a at that point of time immediately after determining that the veneer has a three-dimensional peeling defect 114. Moreover, as indicated above, typically the central parts of a peeler log 100 are in any case usable only as mid-veneers 120₂, whereby,

sometimes the benefits of having veneer without three-dimensional peeling defects formed from core parts of a peeler log, are minute. Most often, in any case, the veneer formed from the core part of the peeler log 100 comprises in any case other types of defects and is thus not usable as a surface veneer sheet 120₁.

As is evident, in order to determine that a surface comprises a three-dimensional peeling defect 114, the surface must first be imaged. Thus, an embodiment of the method comprises imaging also a surface 112 of a secondary veneer 110b, which is free from a peeling defect. Moreover, since the secondary veneer 110b is free from a peeling defect, it is cut to veneer sheets 120. Furthermore, secondary veneer 110b and/or the veneer sheets 120 are dried. Thus, an embodiment comprises imaging also a surface 112 of a secondary veneer 110b, wherein the surface 112 of the secondary veneer 110b is imaged before said drying. By drying the veneer 110b and/or the veneer sheet 120, the surface 112 is also dried. Thus, an embodiment comprises imaging a surface 112 of the secondary veneer 110b with the imaging device 310, before the surface 112 is dried, to form a first signal S₁ indicative of a profile of the surface 112 of the secondary veneer 110b. This has the effect that drying the veneer 110b and/or the veneer sheet 120 typically produces warping to the veneer 110b and/or the veneer sheet. Warping, however, makes the determination of the presence of the peeling defect 114 much harder. It may be hard to detect a protrusion or a depression from a warped surface.

As for the system, in order to ensure that the surface 112 is imaged before drying, in an embodiment, the imaging device 310 is configured to image a surface 112 of the veneer 110 at a distance d₃₁₀ apart from the knife 220 of the veneer lathe 200, wherein the distance d₃₁₀ is small (see Fig. 3a). In an embodiment, the distance d₃₁₀ is at most 5 metres, less than three metres, such as from 1 cm to 2.5 metres. It is also noted that the closer the imaged area is to the knife 220, the more rapidly can the system react to the formation of a peeling defect 114. Therefore, peeling can be stopped before a substantial amount of defected veneer 110 will be produced.

Referring to Figs. 4a and 7c, an embodiment of the method comprises determining a position p₁₁₄ of the peeling defect 114 relative to a longitudinal edge 111 of the primary veneer 110a. The position p₁₁₄ is determined by using the first signal S₁. As indicated in Fig 4a, the longitudinal edge 111 of the primary veneer 110a is such an edge of the primary veneer 110a that extends in the crosswise direction Sy.

In practice, a plywood panel 150 may be finished by sawing the edges of the panel. This may be done before or after sanding, or even if the panel is not sanded. Therefore, it may not be necessary to determine the presence of a such a peeling defect 114 that is very close to the longitudinal edge 111 of the veneer 110. In addition, typically also edges of the veneer 110 are sawn or cut before cutting the veneer 110 into veneer sheets 120. Therefore, it is possible that the second signal S₂ is only generated when a distance p₁₁₄ of the peeling defect 114 from a longitudinal edge 111 exceeds a third limit l₃. The third limit l₃ may be e.g. at least 5 mm or at least 15 mm. Thus, an embodiment comprises determining that the position p₁₁₄ exceeds the third limit l₃.

The determined position p₁₁₄ can also be used e.g. to mark the position of the three-dimensional peeling defect 114. Thus, an embodiment comprises determining a position p₁₁₄ of the peeling defect 114 relative to a longitudinal edge 111 of the primary veneer 110a and marking the peeling defect 114. The peeling defect may be marked with ink or paint. This has the effect that, provided that defected veneer sheets 120 are used as mid-veneer sheets 120₂, the veneer sheets may be more easily classified to mid-veneer sheets 120₂ and to surface veneer sheets 120₁.

## Claims

1. A method for manufacturing a plywood panel (150), the method comprising
- peeling a primary peeler log (100a) with a veneer lathe (200) to form a primary veneer (110a),
- peeling a secondary peeler log (100b) with the veneer lathe (200) to form a secondary veneer (110b),
- cutting the secondary veneer (110b) to form veneer sheets (120),
- drying the secondary veneer (110b) and/or the veneer sheets (120),
- stacking veneer sheets (120) on to each other and arranging adhesive (144) in between veneer sheets (120) to form a veneer sheet stack (130),
- pressing the veneer sheet stack (130) to form the plywood panel (150), and
- imaging a surface (112) of the primary veneer (110a) with an imaging device (310) to form a first signal (S₁) indicative of a profile of the surface (112) of the primary veneer (110a),
**characterized in that** the method comprises
- by using the first signal (S₁), determining that the surface (112) of the primary veneer (110a) has a three-dimensional peeling defect (114) that is a protrusion or a depression and extends in a grain direction (AX) 5 mm to 50 mm and in a crosswise direction (Sy) for more than 50 mm,
wherein the peeler log (100) rotates about a first axis of rotation (AX) and the peeler log (100) is arranged in the veneer lathe (200) such that the grain direction of the peeler log is parallel to the axis of rotation (AX).

2. The method of the claim 1, comprising
- imaging the surface (112) of the primary veneer (110a) with the imaging device (310) at multiple instances of time to form such a first signal (S₁) that is indicative of a profile of the surface of the veneer in two mutually perpendicular dimensions that are perpendicular to a thickness of the primary veneer (110a).

3. The method of claim 1 or 2, comprising
- by using the first signal (S₁), determining that the surface (112) of the primary veneer (110a) has a peeling defect (114) of which height or depth exceeds a first tolerance value (l₁);
preferably,
- the first tolerance value is at least 0.4 mm, such as from 0.4 mm to 1.0 mm, such as from 0.5 mm to 0.8 mm.

4. The method of any of the claims 1 to 3, comprising
- after said pressing the veneer sheet stack (130) to form the plywood panel (150), sanding a surface (152) of the plywood panel (150).

5. The method of claim 4, comprising
- by using the first signal (S₁), determining that the surface (112) of the primary veneer (110a) has a peeling defect (114) of which height or depth exceed a first tolerance value (l₁),
- after said pressing the veneer sheet stack (130) to form the plywood panel (150), sanding a surface (152) of the plywood panel (150) such that at least a sanding thickness (tₛ) is sanded off from the surface (152) of the plywood panel (150), wherein
- the sanding thickness (tₛ) is greater than or equal to the first tolerance value (l₁; ts ≥ l₁).

6. The method of any of the claims 1 to 5, comprising
- after determining that the surface (112) of the primary veneer (110a) has a peeling defect (114),
- stopping peeling the primary peeler log (110a) and cleaning the veneer lathe (200).

7. The method of the claim 6, comprising
- after cleaning the veneer lathe (200),
- peeling the primary peeler log (100a) with the veneer lathe (200) to form subsequent veneer (110),
- cutting the subsequent veneer (110) to form veneer sheets (120),
- drying the subsequent veneer (110) and/or the veneer sheets (120), and
- using the veneer sheets (120) to manufacture the plywood panel (150).

8. The method of claim 6 or 7, comprising
- determining a diameter (di) of the primary peeler log (100a), and
- stopping the peeling of the primary peeler log (100a) only if the diameter (dₗ) of the primary peeler log (100a) exceeds a second tolerance value (l₂).

9. The method of any of the claims 1 to 8, comprising
- imaging a surface (112) of the secondary veneer (110b) with the imaging device (310) to form a first signal (S₁) indicative of a profile of the surface (112) of the secondary veneer (110b), wherein
- the surface (112) of the secondary veneer (110b) is imagined before the surface (112) is dried.

10. The method of any of the claims 1 to 9, comprising
- by using the first signal (S₁), determining a position (p₁₁₄) of the peeling defect (114) relative to a longitudinal edge (111) of the primary veneer (110a); optionally, the method comprises
- determining that the position (p₁₁₄) exceeds a third limit (l₃).

11. A system for manufacturing plywood panels (150), the system comprising
- a veneer lathe (200) comprising
• a rotator (205) configured to rotate the peeler log (100) and
• a knife (220) configured to be set in the rotating peeler log (100) to produce a veneer (110),
- a cutter (400) for cutting the veneer (110) to form veneer sheets (120),
- a dryer (510) for drying the veneer (110) and/or the veneer sheets (120),
- an applicator (520) and a stacker that are, in combination, configured to stack veneer sheets (120) on to each other and apply adhesive (144) in between veneer sheets (120) to form a veneer sheet stack (130),
- a press (530) for pressing the veneer sheet stack (130) to form the plywood panel (150), and
- an imaging device (310) for imaging a surface (112) of the veneer (110), the imaging device (310) configured to form a first signal (S₁) indicative of a profile of the surface (112) of the veneer (110),
**characterized by**
- a processor (380) configured to determine, by using the first signal (S₁), that the surface (112) of the veneer (110) has a three-dimensional peeling defect (114) that is a protrusion or a depression and extends in a grain direction for 5 mm to 50 mm and in a crosswise direction (Sy) for more than 50 mm,
wherein the peeler log (100) rotates about a first axis of rotation (AX) and the peeler log (100) is arranged in the veneer lathe (200) such that the grain direction of the peeler log is parallel to the axis of rotation (AX).

12. The system of claim 11, wherein
- the imaging device (310) is configured to image a surface (112) of the veneer (110) at a distance (d₃₁₀) apart from the knife (220) of the veneer lathe (200), wherein
- the distance (d₃₁₀) is at most 10 metres, preferably at most 5 metres, more preferably less than three metres, such as from 1 cm to 2.5 metres.

13. The system of claim 11 or 12, wherein
- the processor (380) is configured to receive such a first signal (S₁) that is indicative of
• a profile of the surface (112) at multiple instances of time and
• a profile of the surface (112) of the veneer (110) in two mutually perpendicular dimensions that are perpendicular to a thickness of the veneer (110).

14. The system of any of the claims 11 to 13, wherein
- the imaging device (310), such as a camera, is configured to detect light reflected from the surface (112) of the veneer (110) at a first primary directed angle (α₁₁) relative to a first plane (P₁) comprising a normal (N) of the surface (112) of the veneer (110) and a grain direction (AX) of the veneer (100), and the system comprises
- a primary light emitting device (322), configured to emit light to the surface (112) of the veneer (110) at a second primary directed angle (α₁₂) relative to the first plane (P₁) comprising the normal (N) of the surface (112) of the veneer (110) and the grain direction (AX) of the veneer (110).

15. The system of claim 14, wherein
- a difference between the first primary directed angle (α₁₁) and the second primary directed angle (α₁₂) is at least 15 degrees, such as at least 45 degrees; preferably,
- the primary light emitting device (322) comprises a laser.

16. The system of any of the claims 11 to 15, comprising
- a secondary light emitting device (324), configured to emit light to the surface (112) of the veneer (110) at a second secondary angle (α₂₂) relative to a second plane (P₂) comprising a normal (N) of the surface (112) of the veneer (110) and a direction (Sy) that is perpendicular to the grain direction (AX) of the veneer (110), wherein
- the second secondary angle (α₂₂) is at least 30 degrees, such as at least 45 degrees;
preferably,
- the secondary light emitting device (324) comprises a light emitting diode.

17. The system of any of the claims 14 to 16, comprising
- means (330) [A] for keeping the primary (322) and/or the secondary (324) light emitting device clean and/or [B] for cleaning the primary (322) and/or the secondary (324) light emitting device.

18. The system of any of the claims 11 to 17, comprising
- a sander (540) configured to sand a surface (152) of the plywood panel (150).

## Patentansprüche

1. Verfahren zur Herstellung einer Sperrholzplatte (150), wobei das Verfahren Folgendes umfasst:
- Schälen eines primären Schälstammes (100a) mit einer Furnierdrehmaschine (200), um ein Primärfurnier (110a) zu bilden,
- Schälen eines sekundären Schälstammes (100b) mit der Furnierdrehmaschine (200), um ein Sekundärfurnier (110b) zu bilden,
- Schneiden des Sekundärfurniers (110b) zur Bildung von Furnierblättern (120),
- Trocknen des Sekundärfurniers (110b) und/oder der Furnierblätter (120),
- Aufeinanderstapeln der Furnierblätter (120) und Anbringen eines Klebstoffs (144) zwischen den Furnierblättern (120), um einen Furnierblattstapel (130) zu bilden, und
- Pressen des Furnierblattstapels (130) zur Bildung der Sperrholzplatte (150), und
- Abbilden einer Oberfläche (112) des Primärfurniers (110a) mit einer Abbildungsvorrichtung (310), um ein erstes Signal (S₁) zu bilden, welches ein Profil der Oberfläche des Furniers des Primärfurniers (110a) anzeigt,
**dadurch gekennzeichnet, dass** das Verfahren Folgendes umfasst:
- unter Verwendung des ersten Signals (S₁), Feststellen, dass die Oberfläche (112) des Primärfurniers (110a) einen dreidimensionalen Schäldefekt (114) aufweist, der ein Vorsprung oder eine Vertiefung ist und sich in einer Maserungsrichtung (AX) über 5 mm bis 50 mm und in einer Querrichtung (Sy) über mehr als 50 mm erstreckt, wobei der Schälstamm (100) um eine erste Drehachse (AX) rotiert und der Schälstamm (100) in der Furnierdrehmaschine (200) so angeordnet ist, dass die Maserungsrichtung des Schälstammes parallel zu der Drehachse (AX) verläuft.

2. Verfahren nach Anspruch 1, umfassend:
- Abbilden der Oberfläche (112) des Primärfurniers (110a) mit der Abbildungsvorrichtung (310) zu mehreren Zeitpunkten, um ein solches erstes Signal (S₁) zu bilden, welches ein Profil der Oberfläche des Furniers in zwei zueinander senkrechten Abmessungen, die senkrecht zu einer Dicke des Primärfurniers (110a) sind, anzeigen.

3. Verfahren nach Anspruch 1 oder 2, umfassend:
- unter Verwendung des ersten Signals (S₁), Feststellen, dass die Oberfläche (112) des Primärfurniers (110a) einen Schäldefekt (114) aufweist, dessen Höhe oder Tiefe einen ersten Toleranzwert (l₁) überschreitet;
vorzugsweise
- der erste Toleranzwert mindestens 0,4 mm, z. B. 0,4 mm bis 1,0 mm, z. B. 0,5 mm bis 0,8 mm beträgt.

4. Verfahren nach einem der Ansprüche 1 bis 3, umfassend:
- nach dem Pressen des Furnierblattstapels (130) zur Bildung der Sperrholzplatte (150), Schleifen einer Oberfläche (152) der Sperrholzplatte (150).

5. Verfahren nach Anspruch 4, umfassend:
- unter Verwendung des ersten Signals (S₁), Feststellen, dass die Oberfläche (112) des Primärfurniers (110a) einen Schäldefekt (114) aufweist, dessen Höhe oder Tiefe einen ersten Toleranzwert (l₁) überschreitet;
- nach dem Pressen des Furnierblattstapels (130) zur Bildung der Sperrholzplatte (150), Schleifen einer Oberfläche (152) der Sperrholzplatte (150) derart, dass mindestens eine Schleifdicke (tₛ) von der Oberfläche (152) der Sperrholzplatte (150) abgeschliffen wird, wobei
- die Schleifdicke (tₛ) größer als oder gleich dem ersten Toleranzwert (l₁; tₛ ≥ l₁) ist.

6. Verfahren nach einem der Ansprüche 1 bis 5, umfassend:
- nach dem Feststellen, dass die Oberfläche (112) des Primärfurniers (110a) einen Schäldefekt (114) aufweist,
- Anhalten des Schälens des primären Schälstamms (110a) und Reinigen der Furnierdrehmaschine (200).

7. Verfahren nach Anspruch 6, umfassend:
- nach dem Reinigen der Furnierdrehmaschine (200),
- Schälen des primären Schälstamms (100a) mit der Furnierdrehmaschine (200), um das nachfolgende Furnier (110) zu bilden,
- Schneiden des nachfolgenden Furniers (110), um Furnierblätter (120) zu bilden,
- Trocknen des nachfolgenden Furniers (110) und/oder der Furnierblätter (120) und
- Verwenden der Furnierblätter (120) zur Herstellung der Sperrholzplatte (150).

8. Verfahren nach Anspruch 6 oder 7, umfassend;
- Feststellen eines Durchmessers (dₗ) des primären Schälstammes (100a) und
- Anhalten des Schälens des primären Schälstammes (100a) nur dann, wenn der Durchmesser (dₗ) des primären Schälstammes (100a) einen zweiten Toleranzwert (l₂) überschreitet.

9. Verfahren nach einem der Ansprüche 1 bis 8, umfassend:
- Abbilden einer Oberfläche (112) des Sekundärfurniers (110b) mit der Abbildungsvorrichtung (310), um ein erstes Signal (S₁) zu bilden, das ein Profil der Oberfläche (112) des Sekundärfurniers (110b) anzeigt, wobei
- die Oberfläche (112) des Sekundärfurniers (110b) abgebildet wird, bevor die Oberfläche (112) getrocknet ist.

10. Verfahren nach einem der Ansprüche 1 bis 9, umfassend:
- unter Verwendung des ersten Signals (S₁), Feststellen einer Position (p₁₁₄) des Schäldefekts (114) relativ zu einer Längskante (111) des Primärfurniers (110a);
wobei das Verfahren wahlweise umfasst:
- Feststellen, dass die Position (p₁₁₄) eine dritte Grenze (l₃) überschreitet.

11. System zur Herstellung von Sperrholzplatten (150), wobei das System Folgendes umfasst:
- eine Furnierdrehmaschine (200), aufweisend:
• einen Rotator (205), der zum Drehen des Schälstammes (100) konfiguriert ist, und
• ein Messer (220), das so konfiguriert ist, dass es in den rotierenden Schälstamm (100) eingesetzt wird, um ein Furnier (110) herzustellen;
- ein Schneidmesser (400) zum Schneiden des Furniers (110), um Furnierblätter (120) zu bilden,
- einen Trockner (510) zum Trocknen des Furniers (110) und/oder der Furnierblätter (120);
- einen Applikator (520) und einen Stapler, die in Kombination konfiguriert sind, um Furnierblätter (120) aufeinander zu stapeln und Klebstoff (144) zwischen Furnierblättern (120) aufzutragen, um einen Furnierblattstapel (130) zu bilden;
- eine Presse (530) zum Pressen des Furnierblechstapels (130) zur Bildung der Sperrholzplatte (150); und
- eine Abbildungsvorrichtung (310) zum Abbilden einer Oberfläche (112) des Furniers (110), wobei die Abbildungsvorrichtung (310) konfiguriert ist, um ein erstes Signal (S₁) zu bilden, welches ein Profil der Oberfläche (112) des Furniers (110) anzeigt,
**gekennzeichnet durch**
- einen Prozessor (380), der konfiguriert ist, um unter Verwendung des ersten Signals (S₁) festzustellen, dass die Oberfläche (112) des Furniers (110) einen dreidimensionalen Schäldefekt (114) aufweist, der ein Vorsprung oder eine Vertiefung ist und sich in einer Maserungsrichtung (AX) über 5 mm bis 50 mm und in einer Querrichtung (Sy) über mehr als 50 mm erstreckt, wobei der Schälstamm (100) um eine erste Drehachse (AX) rotiert und der Schälstamm (100) in der Furnierdrehmaschine (200) so angeordnet ist, dass die Maserungsrichtung des Schälstammes parallel zur Drehachse (AX) verläuft.

12. System nach Anspruch 11, wobei
- die Abbildungsvorrichtung (310) so konfiguriert ist, dass sie eine Oberfläche (112) des Furniers (110) in einem Abstand (d₃₁₀) zu dem Messer (220) der Furnierdrehmaschine (200) abbildet, wobei
- der Abstand (d₃₁₀) höchstens 10 Meter, vorzugsweise höchstens 5 Meter, besonders bevorzugt weniger als drei Meter, wie beispielsweise von 1 cm bis 2,5 Meter beträgt.

13. System nach Anspruch 11 oder 12, wobei
- der Prozessor (380) so konfiguriert ist, dass er ein solches erstes Signal (S₁) empfängt, das Folgendes anzeigt:
• ein Profil der Oberfläche (112) zu mehreren Zeitpunkten und
• ein Profil der Oberfläche (112) des Furniers (110) in zwei zueinander senkrechten Abmessungen, die senkrecht zu einer Dicke des Furniers (110) sind.

14. System nach einem der Ansprüche 11 bis 13, wobei
- die Abbildungsvorrichtung (310), wie beispielsweise eine Kamera, konfiguriert ist, um von der Oberfläche (112) des Furniers (110) reflektiertes Licht in einem ersten primären gerichteten Winkel (α₁₁) relativ zu einer eine Normale (N) der Oberfläche (112) des Furniers (110) aufweisenden ersten Ebene (P₁) und einer Maserungsrichtung (AX) des Furniers (100) zu erfassen, und das System Folgendes umfasst:
- eine primäre Lichtemissionsvorrichtung (322), die konfiguriert ist, um Licht zur Oberfläche (112) des Furniers (110) in einem zweiten primären gerichteten Winkel (α₁₂) relativ zu der die Normale (N) der Oberfläche (112) des Furniers (110) aufweisenden ersten Ebene (P₁) und der Maserungsrichtung (AX) des Furniers (110) zu emittieren.

15. System nach Anspruch 14, wobei
- ein Unterschied zwischen dem ersten primären gerichteten Winkel (α₁₁) und dem zweiten primären gerichteten Winkel (α₁₂) mindestens 15 Grad, wie z. B. mindestens 45 Grad beträgt, vorzugsweise
- die primäre Lichtemissionsvorrichtung (322) einen Laser aufweist.

16. System nach einem der Ansprüche 11 bis 15, aufweisend:
- eine sekundäre Lichtemissionsvorrichtung (324), die konfiguriert ist, um Licht zur Oberfläche (112) des Furniers (110) unter einem zweiten sekundären Winkel (α₂₂) relativ zu einer eine Normale (N) der Oberfläche (112) des Furniers (110) aufweisenden zweiten Ebene (P₂) und einer Richtung (Sy) zu emittieren, die senkrecht zur Maserungsrichtung (AX) des Furniers (110) ist, wobei
- der zweite sekundäre Winkel (α₂₂) mindestens 30 Grad, wie z. B. mindestens 45 Grad beträgt;
vorzugsweise
- die sekundäre Lichtemissionsvorrichtung (324) eine Leuchtdiode aufweist.

17. System nach einem der Ansprüche 14 bis 16, aufweisend
- Mittel (330) [A] zum Sauberhalten der primären (322) und/oder der sekundären (324) Lichtemissionsvorrichtung und/oder [B] zum Reinigen der primären (322) und/oder der sekundären (324) Lichtem issionsvorrichtung.

18. System nach einem der Ansprüche 11 bis 17, umfassend:
- eine Schleifvorrichtung (540), die zum Schleifen einer Oberfläche (152) der Sperrholzplatte (150) konfiguriert ist.

## Revendications

1. Procédé de fabrication d'un panneau de contreplaqué (150), le procédé comprenant de :
- peler une grume de déroulage primaire (100a) avec une dérouleuse (200) pour former un placage primaire (110a),
- peler une grume de déroulage secondaire (100b) avec la dérouleuse (200) pour former un placage secondaire (110b),
- découper le placage secondaire (110b) pour former des feuilles de placage (120),
- faire sécher le placage secondaire (110b) et/ou les feuilles de placage (120),
- empiler les feuilles de placage (120) les unes sur les autres et disposer un adhésif (144) entre les feuilles de placage (120) pour former une pile de feuilles de placage (130),
- presser la pile de feuilles de placage (130) pour former le panneau de contreplaqué (150), et
- imager une surface (112) du placage primaire (110a) avec un dispositif d'imagerie (310) pour former un premier signal (S₁) indicatif d'un profil de la surface (112) du placage primaire (110a),
**caractérisé en ce que** le procédé comprend de :
- en utilisant le premier signal (S₁), déterminer que la surface (112) du placage primaire (110a) présente un défaut de pelage tridimensionnel (114) qui est une saillie ou une dépression et s'étend dans le sens du fil (AX) pour 5 mm à 50 mm et dans le sens transversal (S_{y}) pour plus de 50 mm,
dans lequel la grume de déroulage (100) tourne autour d'un premier axe de rotation (AX) et la grume de déroulage (100) est disposée dans la dérouleuse (200) de telle sorte que le sens du fil de la grume de déroulage est parallèle à l'axe de rotation (AX).

2. Procédé selon la revendication 1, comprenant de :
- imager la surface (112) du placage primaire (110a) avec le dispositif d'imagerie (310) à plusieurs moments pour former un tel premier signal (S₁) qui est indicatif d'un profil de la surface du placage dans deux dimensions mutuellement perpendiculaires qui sont perpendiculaires à une épaisseur du placage primaire (110a).

3. Procédé selon la revendication 1 ou 2, comprenant de :
- en utilisant le premier signal (S₁), déterminer que la surface (112) du placage primaire (110a) présente un défaut de pelage (114) dont la hauteur ou la profondeur dépasse une première valeur de tolérance (l₁) ;
de préférence,
- la première valeur de tolérance est d'au moins 0,4 mm, par exemple de 0,4 mm à 1,0 mm, par exemple de 0,5 mm à 0,8 mm.

4. Procédé selon l'une quelconque des revendications 1 à 3, comprenant de :
- après ledit pressage de la pile de feuilles de placage (130) pour former le panneau de contreplaqué (150), poncer une surface (152) du panneau de contreplaqué (150).

5. Procédé selon la revendication 4, comprenant de :
- en utilisant le premier signal (S₁), déterminer que la surface (112) du placage primaire (110a) présente un défaut de pelage (114) dont la hauteur ou la profondeur dépasse une première valeur de tolérance (l₁),
- après ledit pressage de la pile de feuilles de placage (130) pour former le panneau de contreplaqué (150), poncer une surface (152) du panneau de contreplaqué (150) de telle sorte qu'au moins une épaisseur de ponçage (tₛ) soit retirée de la surface (152) du panneau de contreplaqué (150), dans lequel
- l'épaisseur de ponçage (tₛ) est supérieure ou égale à la première valeur de tolérance (l₁ ; tₛ ≥ l₁).

6. Procédé selon l'une quelconque des revendications 1 à 5, comprenant de :
- après avoir déterminé que la surface (112) du placage primaire (110a) présente un défaut de pelage (114),
- arrêter le pelage de la grume de déroulage primaire (110a) et nettoyer la dérouleuse (200).

7. Procédé selon la revendication 6, comprenant de :
- après le nettoyage de la dérouleuse (200),
- peler la grume de déroulage primaire (100a) avec la dérouleuse (200) pour former un placage subséquent (110),
- découper le placage subséquent (110) pour former des feuilles de placage (120),
- faire sécher le placage subséquent (110) et/ou les feuilles de placage (120), et
- utiliser les feuilles de placage (120) pour fabriquer le panneau de contreplaqué (150).

8. Procédé selon la revendication 6 ou 7, comprenant de :
- déterminer un diamètre (dₗ) de la grume de déroulage primaire (100a), et
- arrêter le pelage de la grume de déroulage primaire (100a) uniquement si le diamètre (dₗ) de la grume de déroulage primaire (100a) dépasse une deuxième valeur de tolérance (l₂).

9. Procédé selon l'une quelconque des revendications 1 à 8, comprenant de :
- imager une surface (112) du placage secondaire (110b) avec le dispositif d'imagerie (310) pour former un premier signal (S₁) indicatif d'un profil de la surface (112) du placage secondaire (110b), dans lequel
- la surface (112) du placage secondaire (110b) est imaginée avant que la surface (112) ne soit séchée.

10. Procédé selon l'une quelconque des revendications 1 à 9, comprenant de :
- en utilisant le premier signal (S₁), déterminer une position (p₁₁₄) du défaut de pelage (114) par rapport à un bord longitudinal (111) du placage primaire (110a) ;
éventuellement, le procédé comprend de :
- déterminer que la position (p₁₁₄) dépasse une troisième limite (l₃).

11. Système de fabrication de panneaux de contreplaqué (150), le système comprenant :
- une dérouleuse (200) comprenant :
• un rotateur (205) configuré pour faire tourner la grume de déroulage (100) et
• un couteau (220) configuré pour être placé dans la grume de déroulage en rotation (100) pour produire un placage (110),
- un couteau (400) pour découper le placage (110) pour former des feuilles de placage (120),
- un séchoir (510) pour sécher le placage (110) et/ou les feuilles de placage (120),
- un applicateur (520) et un empileur qui sont, en combinaison, configurés pour empiler les feuilles de placage (120) les unes sur les autres et appliquer un adhésif (144) entre les feuilles de placage (120) pour former une pile de feuilles de placage (130),
- une presse (530) pour presser la pile de feuilles de placage (130) pour former le panneau de contreplaqué (150), et
- un dispositif d'imagerie (310) pour imager une surface (112) du placage (110), le dispositif d'imagerie (310) étant configuré pour former un premier signal (S₁) indicatif d'un profil de la surface (112) du placage (110),
**caractérisé par**
- un processeur (380) configuré pour déterminer, en utilisant le premier signal (S₁), que la surface (112) du placage (110) présente un défaut de pelage tridimensionnel (114) qui est une saillie ou une dépression et s'étend dans le sens du fil (AX) pour 5 mm à 50 mm et dans le sens transversal (S_{y}) pour plus de 50 mm,
dans lequel la grume de déroulage (100) tourne autour d'un premier axe de rotation (AX) et la grume de déroulage (100) est disposée dans la dérouleuse (200) de telle sorte que le sens du fil de la grume de déroulage est parallèle à l'axe de rotation (AX).

12. Système selon la revendication 11, dans lequel
- le dispositif d'imagerie (310) est configuré pour imager une surface (112) du placage (110) à une distance (d₃₁₀) du couteau (220) de la dérouleuse (200), dans lequel
- la distance (d₃₁₀) est d'au plus 10 mètres, de préférence d'au plus 5 mètres, plus préférentiellement inférieure à trois mètres, par exemple de 1 cm à 2,5 mètres.

13. Système selon la revendication 11 ou 12, dans lequel
- le processeur (380) est configuré pour recevoir un tel premier signal (S₁) qui est indicatif
• d'un profil de la surface (112) à de multiples moments et
• d'un profil de la surface (112) du placage (110) dans deux dimensions mutuellement perpendiculaires qui sont perpendiculaires à une épaisseur du placage (110).

14. Système selon l'une quelconque des revendications 11 à 13, dans lequel
- le dispositif d'imagerie (310), tel qu'une caméra, est configuré pour détecter la lumière réfléchie par la surface (112) du placage (110) à un premier angle dirigé primaire (α₁₁) par rapport à un premier plan (P₁) comprenant une normale (N) de la surface (112) du placage (110) et un sens du fil (AX) du placage (100), et le système comprend
- un dispositif émetteur de lumière primaire (322), configuré pour émettre une lumière vers la surface (112) du placage (110) à un deuxième angle dirigé primaire (α₁₂) par rapport au premier plan (P₁) comprenant la normale (N) de la surface (112) du placage (110) et le sens du fil (AX) du placage (110).

15. Système selon la revendication 14, dans lequel
- une différence entre le premier angle dirigé primaire (α₁₁) et le deuxième angle dirigé primaire (α₁₂) est d'au moins 15 degrés, par exemple d'au moins 45 degrés ;
de préférence,
- le dispositif émetteur de lumière primaire (322) comprend un laser.

16. Système selon l'une quelconque des revendications 11 à 15, comprenant :
- un dispositif émetteur de lumière secondaire (324), configuré pour émettre une lumière sur la surface (112) du placage (110) à un deuxième angle secondaire (α₂₂) par rapport à un deuxième plan (P₂) comprenant une normale (N) de la surface (112) du placage (110) et une direction (S_{y}) qui est perpendiculaire au sens du fil (AX) du placage (110), dans lequel
- le deuxième angle secondaire (α₂₂) est d'au moins 30 degrés, par exemple d'au moins 45 degrés ;
de préférence,
- le dispositif émetteur de lumière secondaire (324) comprend une diode électroluminescente.

17. Système selon l'une quelconque des revendications 14 à 16, comprenant :
- des moyens (330) [A] pour maintenir propre le dispositif émetteur de lumière primaire (322) et/ou secondaire (324) et/ou [B] pour nettoyer le dispositif émetteur de lumière primaire (322) et/ou secondaire (324).

18. Système selon l'une quelconque des revendications 11 à 17, comprenant :
- une ponceuse (540) configurée pour poncer une surface (152) du panneau de contreplaqué (150).
